# EUROPEAN PATENT APPLICATION

(11) **EP 4 159 202 A1**
(43) Date of publication of application: **05.04.2023**
(21) Application number: 21218273.7
(22) Date of filing: 30.12.2021
(51) Int. Cl.: A61K 9/20, A61K 31/137, A61K 31/4045, A61K 31/675

(54) **PHARMACEUTICAL FORMULATION CONTAINING A PSYCHEDELIC SUBSTANCE OBTAINED BY 3D PRINTING BY SELECTIVE LASER SINTERING (SLS)**

(30) Priority: 29.09.2021 UY 39443; 27.12.2021 US 202163293971 P
(71) Applicant: BIOMIND LABS INC, Toronto, Ontario M5J 2T9 (CA)
(72) Inventor: ANTALICH, Alejandro, Pando, Canelones (UY)
(74) Representative: Padial Martinez, Ana Belen

(57) **Abstract**

The present invention includes a solid oromucosal pharmaceutical form containing a psychedelic selected from psilocybin, psilocin, or mescaline and/or an analog thereof as an active ingredient, a method of preparation of the pharmaceutical form by 3D printing by Selective Laser Sintering (SLS), and treatment of neurological and/or psychiatric disorders, as well as inflammatory disorders.

## Description

### FIELD OF THE INVENTION

The present invention refers to a solid pharmaceutical formulation containing a psychedelic molecule selected from psilocybin, psilocin, or mescaline and/or their analogues as the active ingredient for oromucosal delivery. The formulation is obtained by 3D printing by Selective Laser Sintering (SLS), and can be used in the treatment of neurological and/or psychiatric disorders, as well as various inflammatory disorders.

### SUMMARY

One general aspect of the invention discloses a solid pharmaceutical formulation for oromucosal delivery including i) a psychedelic compound selected from psilocybin, psilocin, mescaline, or an analog thereof; and ii) a thermoplastic polymer. In this embodiment, the solid formulation can be an orodispersible formulation. In this embodiment the solid formulation can be porous.

In an embodiment, the solid pharmaceutical formulation can include 90-95% thermoplastic polymer, 3-5% excipients, and 3-5% of the psychedelic molecule. The thermoplastic polymer can include polyvinylpyrrolidone (PVP), hydroxy propyl methylcellulose (HPMC), polycaprolactone (PCL), poly-L-lactic acid (PLLA), polyethylene (PE), high density polyethylene (HDPE), polyethylene oxide (PEO), and ethyl cellulose (EC).

In an embodiment, the solid pharmaceutical formulation can have a hardness of 2-6 Kg and a friability of less than 1.5.

In an embodiment, the solid pharmaceutical formulation can undergo complete dissolution in less than 10 minutes.

Another general aspect of the invention discloses a method of treating a neurological and/or psychiatric disorder and/or inflammatory disorder by administering to a patient in need thereof the solid pharmaceutical formulation to provide oromucosal delivery.

Another general aspect of the invention discloses a method of making the solid pharmaceutical formulation, the method including:
i) mixing the psychedelic molecule, thermoplastic polymer, and excipients together to form a mixture;
ii) inserting the mixture into the dust bed of a 3D printer; and
iii) 3D printing using Selective Laser Sintering to form the solid pharmaceutical formulation.

### BACKGROUND OF THE INVENTION

Psilocybin, psilocin and mescaline are psychedelic psychotropic substances that cause perceptive changes when consumed.

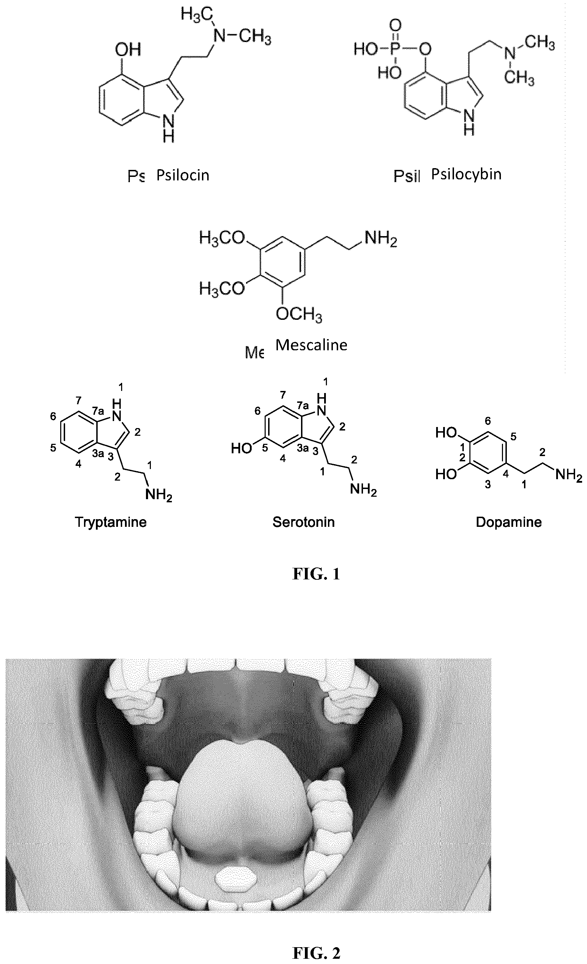

Psilocybin is a natural compound that is found in variable concentrations in more than 200 species of basidiomycete fungi.^{1,2,3} Psilocybin (4-phosphoryl-4-hydroxy-N, N-dimethyltryptamine) and psilocin (4-hydroxy-N, N-dimethyltryptamine) are considered to be tryptamine derivatives (**Figure 1**), due to their central indole ring structure. Psilocybin and psilocin are also very similar to the neurotransmitter serotonin (5-hydroxytryptamine).

Mescaline (3,4,5-trimethoxyphenetylamine) is a phenylethylamine (**Figure 1**), which is mainly found in the Peyote cactus (*Lophophora williamsii*). Structurally, it consists of three methoxide groups attached to a benzene ring in positions 3, 4, and 5, in addition to an aliphatic side chain with an amino group. It has a structural resemblance to the neurotransmitter dopamine.⁴

Tryptamines and phenylethylamines effect the serotonergic and dopamine systems, and, depending on the substance, produce effects in other neurotransmission systems, such as the adrenergic system. These hallucinogens have clinical effects on various biological systems and functions of the body.^{5,6,7} Scientific studies focused on their potential therapeutic uses evidence the benefits of using these compounds to treat various types of ailments and diseases. These molecules have been shown to have a well-established physiological and psychological safety profile in laboratory and clinical research and are non-addictive.⁸

Psilocybin, psilocin and mescalin have been studied by scientists, psychiatrists, and mental health professionals since the 1960s and these and other psychedelics have shown efficacy a promising treatment alternative for a wide range of psychiatric disorders. In recent years, these molecules have been the subject of various preclinical and clinical trials⁹; psilocybin is, to date, the most clinically researched psychedelic. Its potential for the treatment of health problems such as depression, obsessive-compulsive disorder (OCD), anxiety, and addiction to drugs, tobacco and alcohol has been demonstrated. Further, there are studies related to eating disorders, specifically anorexia nervosa.^{10,11,12,13,14,15,16,17}

Another emerging therapeutic area that is very promising for psychedelics is their use as anti-inflammatory agents. Activation of 5-HT2A receptors produces potent anti-inflammatory effects in animal models of human inflammatory disorders at sub-psychedelic levels, i.e. at levels below which psychedelic effects are observed. In general, psychedelics regulate inflammatory pathways through novel mechanisms and represent a new and exciting treatment strategy for various inflammatory disorders.¹⁸

In 2018, the Food and Drug Administration (FDA) designated psilocybin therapy as a "Breakthrough Therapy" for treatment-resistant depressions, and in 2019, for major depressive disorder. Studies have shown that psilocybin is well tolerated and safe for human studies at oral doses of 8 to 25 mg and intravenous doses of 1 to 2 mg.¹⁹

When psilocybin is administered orally or parenterally it dephosphorylates quickly and is almost completely transformed into psilocin.^{20,21} Therefore, psilocin is believed to be responsible for the psychoactive effects. However, when psilocybin is administered orally or parenterally, it is metabolized mainly in the liver, suffering an important first-pass effect, which considerably reduces its concentration before systemic circulation.

When administered intravenously, psilocybin is converted to psilocin in the kidneys, a process that may be less efficient.²²

Pharmacokinetically, significant amounts of psilocin are detectible in plasma within 20 to 40 minutes after oral administration of psilocybin, and maximum concentrations are reached after approximately 80 to 100 minutes.^{23,24,25} Different studies show that psilocybin and psilocin have a plasma elimination half-life of approximately 50 to 160 minutes.^{26,27,28}

Typically, the effects of psilocin disappear completely between 4 and 6 hours after administration.²⁹

Mescaline is rapidly absorbed in the gastrointestinal tract and distributed to the kidneys and liver, where it combines with liver proteins delaying its concentration in the blood. Its metabolization in the body occurs mainly in the liver by the action of an amino oxidase enzyme.^{30,31,32} Orally, the minimum active dose of mescaline is around 100 milligrams. A dose of 500 or 600 milligrams produces a very intense visionary experience, which will last between 6 and 10 hours.

### BRIEF DESCRIPTION OF THE DRAWINGS

Figure 1 shows the atomic diagrams of the structures of psilocybin, psilocin, and mescaline, as well as tryptamine, serotonin, and dopamine.
Figure 2 shows an administration scheme for a 3D-SLS printed oromucosal composition.
Figure 3 shows a graphic scheme of the Selective Laser Sintering (SLS) process.

### DETAILED DESCRIPTION OF THE INVENTION

Oral administration of drugs is preferred by patients for comfort and safety. However, for some drugs, oral administration has a series of disadvantages, including exposure of the active drug to the aggressive gastrointestinal environment which degrades bioactive molecules and limits absorption. This negatively impacts bioavailability and pharmacotherapy. Some of these disadvantages can be partially overcome through parenteral administration, although it is not the preferred choice of patients due to its invasiveness.³³

Transmucosal oral (or oromucosal) administration (use of the mucosa available in the oral cavity) of drugs is a very promising alternative to the oral and parenteral pathways, which avoid some of the disadvantages of oral administration and has several additional advantages. For example, oromucosal delivery reduces first-pass metabolism and thus prevents gastrointestinal degradation. Oromucosal delivery also provides a more direct route to systemic and central nervous system administration resulting in a rapid onset of action of the target drug. It is also a practical and painless route of application, and its use is not yet as widespread in the pharmaceutical industry as other more traditional routes of administration.

In the design of a transmucosal oral formulations there are important aspects to consider, including the need for a very rapid decay of the formulation, and the subsequent dissolution of the drug in a reduced volume of fluid (saliva). These issues are addressed by using compression technology or molding solid formulations with high porosity and utilizing highly effective disintegrants. However, these formulations strategies do not allow precise dosing adjustments according to the needs of patients.³⁴

Oromucosal delivery systems allow for administration of active ingredients with easy absorption by the body, and reduces or avoids metabolism in the liver, and therefore achieves better bioavailability. Oromucosal delivery systems release the active ingredient in the oral cavity and allow for absorption of the active ingredient through the buccal mucosa. Oromucosal delivery systems can also be orodispersible, meaning the delivery system dissolves in the oral cavity. These systems successfully avoid first pass metabolism, which is also known as the first pass effect.

The first pass effect is a phenomenon in which a drug gets metabolized at a specific location in the body that results in a reduced concentration of the active drug upon reaching its site of action or systemic circulation. The first pass effect is often associated with the liver, which is the major site of drug metabolism. However, the first pass effect can also occur in the lungs, vasculature, gastrointestinal tract, and other metabolically active tissues in the body. The effect can also be augmented by various factors, including plasma protein concentrations, enzymatic activity, and gastrointestinal motility.

Drug delivery via the oral mucous membranes is an attractive, alternative to other oral administration methods win which the active ends up in the gastrointestinal tract offering several advantages. In particular, oromusocal administration delivers the active drug via the sublingual mucosa (i.e., the membrane of the ventral surface of the tongue and the floor of the mouth) where it is absorbed and enters into systemic circulation while bypassing the gastrointestinal tract. This avoids first pass effects and results in improved bioavailability, rapid onset of drug action, improved patient compliance, as well as complete avoidance of dysphagia (i.e., difficulty in swallowing).

Use of 3D printing is a novel and emerging technological application within the pharmaceutical industry that has the potential to solve issues commonly associated with transmucosal formulation.

Additive manufacturing by 3D printing, is a layer-by-layer manufacturing process that allows three-dimensional pharmaceutical forms to be produced from digital designs. There are numerous processes adaptable to additive manufacturing, with Selective Laser Sintering (SLS) being one of the most promising.³⁵

The SLS technique can be used to obtain highly porous pharmaceutical forms with very low decay times, which is ideal for oromucosal administration via the sublingual route (Figure 2). Using this technique results in a rapid onset of action and a low hepatic first step effect. Additionally, printing software can be modified to obtain high-dose formulations and different controlled-release particles can be printed to modify and adapt the dosage in real time or personalized to a particular subject.

Macro and microstructure conformations can be printed to adjust the dissolution profile of the active ingredients and allows for the design of flexible dosage forms for personalized medicine.^{36,37,38}

US 5,490,962 describes the preparation of dosage forms using Solid Freeform Fabrication (SFF) methods. These methods can be adapted to be used with a variety of different materials to create dosage forms with defined compositions, forces, and densities. The SLS method is an example of the SFF method and allows for manipulation of the macrostructure and porosity of the dosage forms by manipulating the 3D printing parameters, the type of polymer, and the size of the particles, as well as the solvent and / or ligand. WO98/36739 references US 5,490,962, emphasizing that in certain therapies, a pulsed release of drugs for long periods of time is required. Therein a multiphase dosing method is described, which provides a differential release of the different drugs through 3D printing.

US 2019/0374471 and US 2021/0169811 discuss the use of 3D printing by SLS to obtain a wide variety of formulations (capsules, films, patches) pre-loaded with a drug with a wide range of dissolution averages and decay speeds that depend on the composition of the formulations. US 10,350,822 and WO 2020/148442 disclose oral dosing forms composed of multiple layers of a material mixed with an active ingredient of different chemical nature that are obtained by 3D printing. This type of technology allows for a drug release profile according to the needs of the therapy.

Using SLS 3D printing allows for improved safety, efficacy, and accessibility of medicines. It also allows for the personalized selection of other formulation components such as excipients, design, and color.

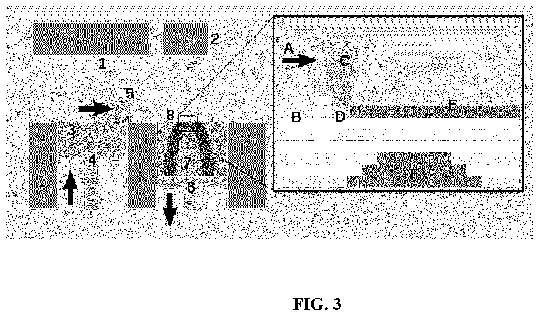

Disclosed herein are pharmaceutical formulations for oromucosal delivery obtained by 3D printing by Selective Laser Sintering (SLS) (Figure 3) containing a psychedelic molecule selected from psilocybin, psilocin and/or mescaline and/or their analogues as the active ingredient. As shown in Figure 3, the SLS 3D printing system uses a Laser (1) coupled to a scanning system (2), A dust dispensing system (3) distributes the reactive dust that includes the drug and a thermoplastic polymer. The distributions system expels the dust using a dust dispensing piston (4) with the dust evenly distributed on a surface using a roller (5). A manufacturing piston (6) lowers a manufacturing platform (7), which can hold a solid pharmaceutical form or mold (8) during in printing process. Figure 3 also shows the Laser scanning direction (A), Sintered dust particles (B), Laser beam (C), Laser sintering of dust particles (D), dust particles pre-positioned for sintering (E), and the pharmaceutical mold or form (F).

Conventional pharmaceutical formulation technology does not allow for modification of dosage formulations in real time. Complex changes to manufacturing equipment are necessary to change simple characteristics, such as geometry and color, among others.

Compared with conventional formulation techniques, SLS 3D printing has competitive advantages that improve the safety, effectiveness, and accessibility of medicines by allowing for the personalized selection of formulation components, such as excipients, design, and color.

This technology also allows for the development of complex products, products on demand and / or personalized products. For example, geometry (shape) and/or color can be readily changed in SLS 3D printing to increase adherence to dosing regimens for marketing and treatment purposes.

In the development and production of personalized medicines, SLS 3D printing allows for the proper selection of manufacturing parameters. For example, tablets can be produced with the exact dose of the active ingredient that suits the individual needs of each patient. SLS 3D printing also allows for the design of a pharmaceutical formulation with controlled time release of the drug it contains and, in this way, increase its action time. Geometries can also be varied (for example by changing thickness, layering and surface area) or components selected to alter release characteristics and other properties of the formulations. Release characteristics can include, for example, the rate of release of the active, the duration of release, and the total amount of active delivered.

The SLS 3D printing process consists of a laser that sweeps the surface of a bed of powder to sinter or melt it. The bed of dust is then displaced downwards, new dust is distributed above it, and the process is repeated to produce a solid object.

Sintering generally occurs at relatively high temperatures, limiting its applicability to active ingredient that can survive the process without degradation. With selection of appropriate polymers, psilocybin, psilocin, and mescaline can withstand the high sintering temperatures.

There are pharmaceutical compositions known in the art containing at least one of the psychedelic molecules psilocybin, psilocin and/or mescaline, and strategies to avoid first pass metabolism of the psychedelic. However, there is no disclosure that proposes a formulation suitable for oromocusal administration, nor use of SLS 3D printing to manufacture the oromucosal formulation.

Disclosed herein is the 3D printing SLS production of a pharmaceutical formulation for oromucosal delivery containing one of the following psychedelic molecules, psilocybin, psilocin, or mescaline as an active ingredient.

Herein, a solid oromucosal pharmaceutical formulation is disclosed containing any of the following psychedelic molecules, psilocybin, psilocin, or mescaline and / or their analogues, as an active ingredient, and the formulations use in the treatment of neurological and/or psychiatric disorders, as well as an anti-inflammatory agent for various inflammatory disorders. The formulations can be manufactured to have a higher porosity. The formulations can also be manufactured to have high-water capture. The high-water capture is achieved by including using hydrophilic polymers and/or excipients in the formulation. These hydrophilic polymers or excipients quickly capture water from the saliva to promote rapid dissolution of the formulation for oromucosal delivery.

In exemplary embodiments, the material is sintered at a temperature of between 90 and 130 degrees Celsius, and the material contains 3-5% of the psychedelic active ingredient, 90-95% of thermoplastic polymer, and 0-5%, for example 3-5% of additional excipients. A solid pharmaceutical formulation is obtained with appropriate mechanical properties for packaging and administration. For example, the formulation can have a hardness of 2-6 kg and a friability of less than 1.5. The solid oromucosal delivery formulation undergoes complete dissolution in less than 10 minutes.

Suitable thermoplastic polymers for use in the formulations include, but are not limited to, polyvinylpyrrolidone (PVP), hydroxy propyl methylcellulose (HPMC), polycaprolactone (PCL), poly-L-lactic acid (PLLA), polyethylene (PE), high density polyethylene (HDPE), polyethylene oxide (PEO), and ethyl cellulose (EC), among others. In exemplary embodiments, the thermoplastic polymer is hydrophilic. Other hydrophilic excipients, such as binders and dispersants, are known in the art and can be suitably selected by the skilled artisan to provide the desired solubility and water capture properties.

The invention is exemplified in more detail in the two non-limiting examples below.

### EXAMPLE 1

A psychedelic molecule (psilocybin, psilocin or mescaline and/or their analogues), a thermoplastic polymer and, optionally, other the excipients are passed through a 100-mesh sieve and ground if necessary. Then all the ingredients are mixed for 15 minutes. They are then inserted into the dust bed of the 3D printer, with a distance of 1.0 mm between the laser and powder bed. Then, the printing platform is moved along the Z axis. Excess dust is removed using a device situated along the X axis. Laser printing is done using a printer equipped with a 450 nm laser generator.

The prints are designed using specific software and the laser power is set to 0.05 - 3.5 W with a print depth (i.e., sintering depth) of between 3% - 80%.

The thickness is measured in the center or at the edge using a micrometer and then mechanical tests for hardness and friability are performed.

### EXAMPLE 2

The psychedelic molecules (psilocybin, psilocin, or mescaline and/or their analogues) are mixed together with the inactive materials (polymers and excipients) in a double cone mixer for 20 minutes.

The forms are designed with Autodesk Inventor 2020 and saved in the STL file format. Printing is done using a 2.3 W blue laser printer (445 nm wavelength).

The temperature of the internal printing camera and the temperature of the dust surface is experimentally adjusted, and the laser scanning speed is set at 100 mm/s. The thickness of the layer of dust is set to 100 µm.

Compact prints are obtained at an internal chamber temperature of 140°C and a dust surface temperature of 155°C.

The thickness is measured in the center or at the edge using a micrometer and then mechanical tests for hardness and friability are performed.

### REFERENCES

1 Berger K.J. and Guss D.A., The Journal of Emergency Medicine, 2005; 28(2):175-183: "Mycotoxins revisited: Part II".
2 Guzman, G., Economic Botany, 2008, 62(3):404-412: "Hallucinogenic Mushrooms in Mexico: An Overview".
3 Burillo-Putze G, et al., Anales del Sistema Sanitario de Navarra, 2013, 36(3):505-518: "Drogas emergentes (III): plantas y hongos alucinógenos".
4 Kulma A. and Szopa J., Plant Science, 2007;172(3):433-440: "Catecholamines are active compounds in plants".
5 Fantegrossi W.E. et al., Pharmacology, biochemistry, and behavior, 2008, 88:358-365: "Hallucinogen-like effects of N,N-dipropyltryptamine (DPT): possible mediation by serotonin 5HT1A and 5-HT2A receptors in rodents".
6 McKenna D.J. et al., Neuropharmacology, 1990, 29:193-198: "Differential interactions of indolealkylamines with 5-ydroxytryptamine receptor subtypes.
7 Rickli A. et al., European neuropsychopharmacology, 2016, 26:1327-1337: "Receptor interaction profiles of novel psychoactive tryptamines compared with classic hallucinogens".
88 Johnson M., et al., Journal of Psychopharmacology, 2008, 22:603-620: "Human hallucinogen research: guidelines for safety".
9 Kyzar et al., Trends in Pharmacological Science, 2017, 38(11):992-1005: "Review Psychedelic Drugs in Biomedicine".
10 de Veen B.T. et al., Expert Review of Neurotherapeutics, 2016, 17:203-212: "Psilocybin for treating substance use disorders".
11 ; Ross S., Psychiatric Clinics of North America, 2012, 35(2):357-374: "Serotonergic hallucinogens and emerging targets for addiction pharmacotherapies".
12 Johnson M.W. et al., Journal of Psychopharmacology, 2012, 28(11):983-992: "Pilot study of the 5-HT2AR agonist psilocybin in the treatment of tobacco addiction".
13 Grob C.S. et al., Archives of General Psychiatry, 2011, 68:71-78: "Pilot-study of psilocybin treatment for anxiety in patients with advanced-stage cancer".
1414 Griffiths R.R. et al., Journal of Psychopharmacology, 2016, 28:1181-1197: "Psilocybin produces substantial and sustained decreases in depression and anxiety in patients with life-threatening cancer: a randomized double-blind trial".
15 Ross S. et al., Journal of Psychopharmacology, 2016, 30:1165-1180: "Rapid and sustained symptom reduction following psilocybin treatment for anxiety and depression in patients with life threatening cancer: a randomized controlled trial".
16 Carhart-Harris R.L. et al., Lancet Psychiatry, 2016, 3:619-627: "Psilocybin with psychological support for treatment-resistant depression: an open-label feasibility study".
17 Winkelman M., Current drug abuse reviews, 2014, 72:10116: Psychedelics as medicines for substance abuse rehabilitation: evaluating treatments with LSD, Peyote, Ibogaine and Ayahuasca".
18 Flanagan T.W. and Nichols C.D., International Review of Psychiatry, 2018, "Psychedelics as anti-inflammatory agents".
19 Passie T. et al., Addiction Biology, 2002, 7:357-364: "The pharmacology of psilocybin".
2020 Horita A., Weber LJ., Biochemical Pharmacology, 1961, 7:47-54: "The enzymic dephosphorylation and oxidation of psilocybin and psilocin by mammalian tissue homogenates".
21 Hasler F. et al., Pharmaceutica acta Helvetiae, 1997, 72:175-184: "Determination of psilocin and 4-hydroxyindole-3-acetic acid in plasma by HPLC-ECD and pharmacokinetic profiles of oral and intravenous psilocybin in man".
22 Hasler F., et al., Journal of pharmaceutical and biomedical analysis, 2002, 30:331-339: "Renal excretion profiles of psilocin following oral administration of psilocybin: a controlled study in man".
23 Hasler F. et al., Pharmaceutica acta Helvetiae, 1997, 72:175-184: "Determination of psilocin and 4-hydroxyindole-3-acetic acid in plasma by HPLC-ECD and pharmacokinetic profiles of oral and intravenous psilocybin in man".
24 Lindenblatt H., et al., Journal of chromatography. B, Biomedical sciences and applications, 1998, 709:225-263: "Quantitation of psilocin in human plasma by high performance liquid chromatography and electrochemical detection: comparison of liquid-liquid extraction with automated on-line solid-phase extraction".
25 Passie T., et al., Addiction Biology, 2002, 7:357-364: "The pharmacology of psilocybin".
26 Hasler F., et al., Pharmaceutica acta Helvetiae, 1997, 72:175-184: "Determination of psilocin and 4-hydroxyindole-3-acetic acid in plasma by HPLC-ECD and pharmacokinetic profiles of oral and intravenous psilocybin in man".
27 Martin R, et al., International journal of legal medicine, 2013b, 127:593-601: "Determination of psilocin, bufotenine, LSD and its metabolites in serum, plasma and urine by SPE-LC-MS/MS".
28 Martin R., et al., International journal of legal medicine, 2012, 126:845-849: "A validated method for quantitation of psilocin in plasma by LC-MS/MS and study of stability".
29 Shulgin A.T., Journal of psychedelic drugs, 1980, 12:79: "Profiles of psychedelic drugs. Psilocybin" .
3030 Kapadia G.J. and Fayez M.B., Journal of pharmaceutical sciences, 1970, 59(12):1699-1727: "Peyote constituents: chemistry, biogenesis, and biological effects".
31 Dasgupta A., Advances in clinical chemistry, 2017, 78:163-186: "Challenges in Laboratory Detection of Unusual Substance Abuse: Issues with Magic Mushroom, Peyote Cactus, Khat, and Solvent Abuse".
3232 Hilliker K.S. and Roth R.A., Biochemical Pharmacology, 1980, 29(2):253-255: "Prediction of mescaline clearance by rabbit lung and liver from enzyme kinetic data".
33 Dubey, S.K. et al., Drug Discovery Today, 2021, 26(4):931-950: "Oral peptide delivery: challenges and the way ahead".
34 Mathias N.R. et al., Journal of Pharmaceutical Sciences, 2010, 99(1):1-20: "Non-invasive Systemic Drug Delivery: Developability Considerations for Alternate Routes of Administration".
35 Kulinowski, P. et al., Additive Manufacturing, 2021, 38:101761: "Selective laser sintering (SLS) technique for pharmaceutical applications Development of high dose controlled release printlets".
36 Awad A. et al., International Journal of Pharmaceutics, 2020, 586:119594: "3D printing: Principles and pharmaceutical applications of selective laser sintering".
37 Fina, F. et al., International Journal of Pharmaceutics, 2018, 547(1-2):44-52: "3D printing of drug-loaded gyroid lattices using selective laser sintering".
38 Thakkar R. et al., European Journal of Pharmaceutics and Biopharmaceutics, 2021, 163:141-156: "Synergistic application of twin-screw granulation and selective laser sintering 3D printing for the development of pharmaceutical dosage forms with enhanced dissolution rates and physical properties".

## Claims

1. A solid pharmaceutical formulation for oromucosal delivery comprising i) a psychedelic compound selected from the group consisting of psilocybin, psilocin, mescaline, or an analog thereof; and ii) a thermoplastic polymer.

2. The solid pharmaceutical formulation of claim 1, wherein the formulation is an orodispersible.

3. The solid pharmaceutical formulation of claim 1, wherein the formulation is porous.

4. The solid pharmaceutical formulation of claim 1, wherein the formulation comprises 90-95% thermoplastic polymer.

5. The solid pharmaceutical formulation of claim 1, wherein the formulation further comprises 3-5% excipients.

6. The solid pharmaceutical formulation of claim 1, wherein the formulation comprises 3-5% of the psychedelic molecule.

7. The solid pharmaceutical formulation of claim 1, wherein the formulation has a hardness of 2-6 Kg and a friability of less than 1.5

8. The solid pharmaceutical formulation according to claim 1, wherein the formulation undergoes complete dissolution in less than 10 minutes.

9. The solid pharmaceutical formulation according to claim 1, wherein the thermoplastic polymer is selected from the group consisting of polyvinylpyrrolidone (PVP), hydroxy propyl methylcellulose (HPMC), polycaprolactone (PCL), poly-L-lactic acid (PLLA), polyethylene (PE), high density polyethylene (HDPE), polyethylene oxide (PEO), and ethyl cellulose (EC).

10. A method of treating a neurological and/or psychiatric disorder and/or inflammatory disorder comprising administering to a patient in need thereof the solid pharmaceutical formulation of claim 1 to provide oromucosal delivery.

11. A method of making the solid pharmaceutical formulation of claim 1, the method comprising:
i) mixing the psychedelic molecule, thermoplastic polymer, and excipients together to form a mixture;
ii) inserting the mixture into the dust bed of a 3D printer; and
iii) 3D printing using Selective Laser Sintering to form the solid pharmaceutical formulation.
